# EUROPEAN PATENT APPLICATION

(11) **EP 2 186 787 A1**
(43) Date of publication of application: **19.05.2010**
(21) Application number: 08253723.4
(22) Date of filing: 13.11.2008
(51) Int. Cl.: C07C 29/149, C07C 31/04, C07C 31/08, C07C 51/12, C07C 53/08

(54) **Hydrogenation of ethanoic acid to produce ethanol**

(71) Applicant: BP p.l.c., London SW1Y 4PD (GB)
(72) Inventor: The designation of the inventor has not yet been filed
(74) Representative: De Kezel, Eric

(57) **Abstract**

The present invention relates to a process for the production of ethanol (and optionally methanol) from a carbonaceous feedstock; wherein the ethanoic acid (produced by a multi-step process from the carbonaceous feedstock) is hydrogenated to produce ethanol in the same reactor unit and with the same catalyst(s) that is used to produce methanol from synthesis gas.

## Description

The present invention relates to the production of ethanol (and optionally methanol) from ethanoic acid.

The present invention relates to a process for the production of ethanol (and optionally methanol) from a carbonaceous feedstock; wherein.the ethanoic acid (produced by a multi-step process from the carbonaceous feedstock) is hydrogenated to produce ethanol within the same alcohol synthesis unit and in the presence of the same catalyst(s) that is used to produce the said methanol from the said synthesis gas.

In recent years increased use and demand for alcohols such as methanol, ethanol and higher alcohols has led to a greater interest in processes relating to alcohol production. The said alcohols may be produced by the fermentation of, for example, sugars and/or cellulosic materials.

Alternatively alcohols, such as ethanol, may be produced from synthesis gas. Synthesis gas refers to a combination of H₂ and carbon oxides produced in a synthesis gas plant from a carbon source such as natural gas, petroleum liquids, biomass and other carbonaceous materials including coal, recycled plastics, municipal wastes, or any organic material. Thus, alcohol and alcohol derivatives may provide non-petroleum based routes for the production of valuable chemicals and fuels.

Generally, the production of alcohols, for example methanol, takes place via three process steps: synthesis gas preparation, methanol synthesis, and methanol purification. In the synthesis gas preparation step, an additional stage may be employed whereby the feedstock is treated, e.g. the feedstock is purified to remove sulphur and other potential catalyst poisons prior to being converted into synthesis gas. This treatment can also be conducted after synthesis gas preparation; for example, when coal or biomass is employed.

The reaction to produce alcohol(s) from synthesis gas is generally exothermic. The formation of C₂ and C₂₊ alcohols is believed to proceed via the formation of methanol for modified methanol catalysts and cobalt molybdenum sulphide catalysts. However, the production of methanol is equilibrium-limited and thus requires high pressures in order to achieve viable yields. Hence, pressure can be used to increase the yield, as the reaction which produces methanol exhibits a decrease in volume, as disclosed in US 3326956.

A low-pressure, copper-based methanol synthesis catalyst is commercially available from suppliers such as BASF, Johnson Matthey, and Haldor-Topsoe. Methanol yields from copper-based catalysts are generally over 99.5% of the converted CO+CO₂ present. Water is a by-product of the conversion of CO₂ to methanol and the conversion of CO synthesis gas to C₂ and C₂₊ oxygenates. In the presence of an active water-gas shift catalyst, such as a methanol catalyst or a cobalt molybdenum catalyst the water equilibrates with the CO to give CO₂ and H₂. A paper entitled, "Selection of Technology for Large Methanol Plants," by Helge Holm-Larsen, presented at the 1994 World Methanol Conference, Nov. 30-Dec. 1, 1994, in Geneva, Switzerland, reviews the developments in methanol production and shows how further reduction in costs of methanol production will result in the construction of very large plants with capacities approaching 10,000 t per day.

Other processes for the production of C₂ and C₂₊ alcohol(s), include the processes described hereinafter;

WO 8303409 describes a process whereby ethanol is produced by carbonylation of methanol by reaction with CO in the presence of a carbonylation catalyst to form ethanoic acid which is then converted to an ethanoate ester followed by hydrogenolysis of the ethanoate ester formed to give ethanol or a mixture of ethanol and another alcohol which can be separated by distillation. Carbonylation can be effected using a CO/H₂ mixture and hydrogenolysis can similarly be conducted in the presence of CO, leading to the possibility of circulating gas between the carbonylation and hydrogenolysis zones with synthesis gas, preferably a 2:1 H₂:CO molar mixture being used as make up gas.

US 4122110 relates to a process for manufacturing alcohols, particularly linear saturated primary alcohols, by reacting CO with H₂ at a pressure between 2 and 25 MPa and a temperature between 150 and 400 °C, in the presence of a catalyst, **characterized in that** the catalyst contains at least 4 essential elements: (a) copper (b) cobalt (c) at least one element M selected from chromium, iron, vanadium and manganese, and (d) at least one alkali metal.

US 4831060 relates to the production of mixed alcohols from CO and H₂ gases using a catalyst, with optionally a co-catalyst, wherein the catalyst metals are molybdenum, tungsten or rhenium, and the co-catalyst metals are cobalt, nickel or iron. The catalyst is promoted with a Fischer-Tropsch promoter like an alkali or alkaline earth series metal or a smaller amount of thorium and is further treated by sulphiding. The composition of the mixed alcohols fraction can be selected by selecting the extent of intimate contact among the catalytic components.

Journal of Catalysis, 1988, 114, 90-99 discloses a mechanism of ethanol formation from synthesis gas over CuO/ZnO/Al₂O₃. The formation of ethanol from CO and H₂ over a CuO/ZnO methanol catalyst is studied in a fixed-bed microreactor by measuring the isotopic distribution of the carbon in the product ethanol when isotopically-enriched ¹³C methanol was added to the feed.

As the importance of ethanol is ever increasing in today's world, so is the need and desire to produce ethanol from a carbonaceous feedstock with a higher carbon efficiency, a higher conversion and an improved productivity and selectivity. Hence, the present invention provides a process that allows one to produce ethanol from a carbonaceous feedstock, with an improved carbon efficiency, a higher selectivity and, in particular, with a more productive conversion to ethanol.

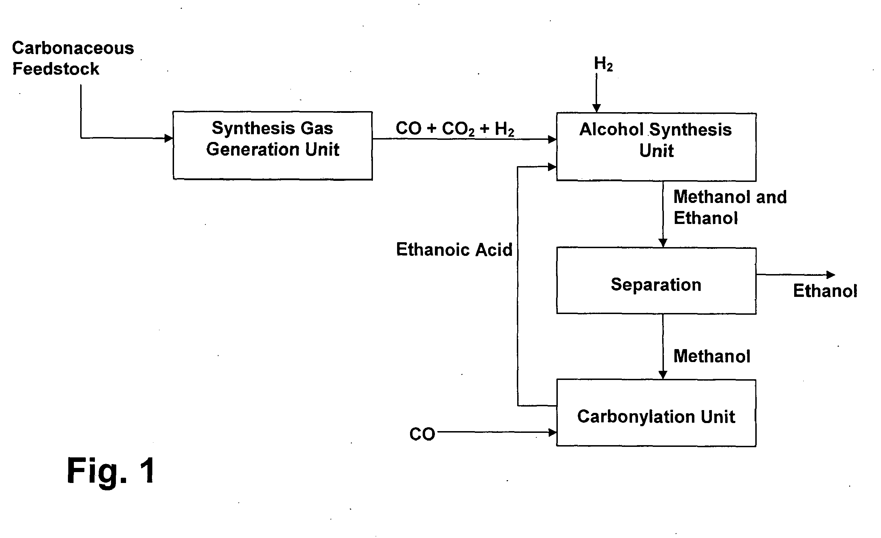

Figure 1 represents an embodiment of a process scheme according to the present invention, wherein the references correspond to those used in the present description and appending claims.

Thus, the present invention relates to a process for producing methanol and ethanol, wherein the methanol is produced from synthesis gas and the ethanol is produced via the hydrogenation of an ethanoic acid feed; **characterised in that** the hydrogenation of the ethanoic acid feed is carried out with the same catalyst(s), and within the same alcohol synthesis unit, that is used to produce the said methanol from the said synthesis gas.

Also the present invention relates to a process for the conversion of synthesis gas to ethanol, characterised by the following steps:
1) introducing synthesis gas, together with ethanoic acid, into an alcohol synthesis unit to produce methanol and ethanol,
2) separating the methanol from the ethanol of step 1,
3) introducing methanol, from step 2, together with CO, into a carbonylation reactor in the presence of a methanol carbonylation catalyst, to produce ethanoic acid,
4) feeding ethanoic acid, produced in step 3, into the alcohol synthesis unit of step 1, and
5) recovering ethanol from step 2.

Furthermore, the present invention relates to a process for the conversion of a carbonaceous feedstock(s) into ethanol, characterised by the following steps:
1) introducing a carbonaceous feedstock into a synthesis gas reactor to produce a mixture of carbon oxide(s) and H₂,
2) introducing CO and H₂, from step 1, together with ethanoic acid, into an alcohol synthesis unit to produce methanol and ethanol,
3) separating the methanol from the ethanol of step 2,
4) introducing methanol, from step 3, together with CO, into a carbonylation reactor in the presence of a methanol carbonylation catalyst, to produce ethanoic acid,
5) feeding ethanoic acid, produced in step 4, into the alcohol synthesis unit of step 2, and
6) recovering ethanol from step 3.

For the purposes of the present invention and appending claims the following terms are defined hereinafter:
- The 'dew point temperature' is a threshold temperature, for example, for a given pure component or mixture of components, at a given pressure, if the system temperature is raised to *above* the dew point temperature, the mixture will exist as a dry gas. Likewise *below* the dew point temperature, the mixture will exist as a vapour containing some liquid.
- 'Gas' and/or 'gas phase' are defined as a pure component, or mixture of components, that are above the dew point temperature.
- 'Gas hourly space velocity' (GHSV) is defined as the volume of gas fed per unit volume of catalyst per hour, at standard temperature (0 °C) and pressure (0.101325 MPa).
- 'Liquid hourly space velocity' (LHSV) is defined as the volume of liquid fed per unit volume of catalyst per hour.

According to one aspect of the present invention, the synthesis gas feedstock, a mixture of carbon oxide(s) and H₂, that is used to produce the methanol feed stream, is preferably produced from a carbonaceous feedstock.

The carbonaceous feedstock is preferably a material such as biomass, plastic, naphtha, refinery bottoms, crude synthesis gas (from underground coal gasification or biomass gasification), smelter off gas, municipal waste, coal bed methane, coal, and/or natural gas, with coal and natural gas being the preferred sources. To one skilled in the art a combination of sources can also be used, for example coal and natural gas to advantageously increase the H₂ to carbon ratio.

Natural gas commonly contains a range of hydrocarbons (e.g. C₁-C₃ alkanes), in which methane predominates. In addition to this, natural gas will usually contain nitrogen, CO₂ and sulphur compounds. Preferably the nitrogen content of the feedstock is less than 40 mol %, more preferably less than 10 mol % and most preferably less than 2 mol %.

Processes for producing synthesis gas, in a synthesis gas plant, are well known. Each method has its advantages and disadvantages, and the choice of using a particular reforming process over another is governed by economic and available feed stream considerations, as well as by the desire to obtain the optimum (H₂-CO₂):(CO+CO₂) molar ratio in the resulting synthesis gas that is suitable for further chemical processing. A discussion of the available synthesis gas production technologies is provided in both Hydrocarbon Processing, 1999, 78:4, 87-90, and 92-93 and Petrole et Techniques, 1998, 415, 86-93, and are both hereby incorporated by reference.

It is also known that the synthesis gas may be obtained by catalytic partial oxidation of hydrocarbonaceous material in a microstructured reactor as exemplified in IMRET 3: Proceedings of the Third International Conference on Microreaction Technology, ed. W. Ehrfeld, Springer Verlag, 1999, pages 187-196. Alternatively, the synthesis gas may be obtained by short contact time catalytic partial oxidation of hydrocarbonaceous feedstocks as described in EP 0303438. The synthesis gas can also be obtained via a 'compact reformer' process as described in Hydrocarbon Engineering, 2000, 5:5, 67-69; Hydrocarbon Processing, 2000, 79:9, 34; Today's Refinery, 2000, 15:8, 9; WO 9902254; and WO 0023689.

Typically, for commercial synthesis gas production the pressure at which the synthesis gas is produced from a steam reformer ranges from approximately 0.1 to 10 MPa, preferably 2 to 3 MPa and the temperatures at which the synthesis gas exits the reformer ranges from approximately 700 to 1000°C. Likewise, for commercial synthesis gas production the pressure at which the synthesis gas is produced from an auto-thermal reformer ranges from approximately 0.1 to 10 MPa, preferably 2 to 5 MPa and the temperatures at which the synthesis gas exits the reformer ranges from approximately 700 to 1300 °C. Where the high temperatures are necessary in order to produce a favourable equilibrium for synthesis gas production, and to avoid metallurgy problems associated with carbon dusting. The synthesis gas contains a molar ratio of (H₂-CO₂):(CO+CO₂) ranging from 0.8 to 3.0, which is dependent on the carbonaceous feedstock(s) and the method of reforming used. For example, when natural gas is used as the carbonaceous feedstock for steam reforming, the synthesis gas obtained usually has a maximum (H₂-CO₂):(CO+CO₂) ratio of 3.0. However, when natural gas is used as the carbonaceous feedstock for auto-thermal reforming, the synthesis gas obtained usually has a (H₂-CO₂):(CO+CO₂) ratio of 1.5.

According to a preferred embodiment of the present invention, the molar ratio, (H₂-CO₂):(CO+CO₂), of the synthesis gas stream exiting the synthesis gas generation unit(s) is greater than 1.6, more preferably greater than 1.8 and most preferably greater than 2.0. Preferably, the molar ratio, (H₂-CO₂):(CO+CO₂), of said synthesis gas stream exiting the synthesis gas generation unit(s) is less than 3.0, preferably less than 2.75, more preferably less than 2.4 and most preferably less than 2.2.

According to another embodiment of this invention when the carbonaceous feedstock used for synthesis gas generation is not an aliphatic hydrocarbon (e.g. coal, aromatic material, biomass) the molar ratio (H₂-CO₂):(CO+CO₂) of the exit synthesis gas is preferably adjusted to the target value by the addition of H₂ or the removal of CO₂.

CO₂ may be removed by the use of a simple, yet effective, separation method known to those skilled in the art, for example, a "membrane separation method". Such membrane technologies can be found in 'Purification and Recovery Options for Gasification' D. J. Kubek, E. Polla, F. P. Wilcher, UOP, 1996.

Alternatively, CO₂ may be recovered and removed by any suitable method(s) known to those skilled in the art, for example, by reacting with amines; performing a methanol wash (i.e. the RECTISOL process) and/or by using hot potassium carbonate (e.g. the BENFIELD process).

According to a preferred embodiment of the present invention, the exit stream obtained from the synthesis gas reactor (e.g. using a steam reformer), comprises essentially a mixture of carbon oxide(s) and H₂. It can also comprise water, nitrogen and traces of unconverted hydrocarbons (e.g. C₁-C₃ alkanes).

According to a preferred embodiment of the present invention, during synthesis gas generation, an additional stage may be employed whereby the feedstock is first purified to remove sulphur and other potential catalyst poisons (such as halides or metals e.g. mercury) prior to being converted into synthesis gas; alternatively this treatment can also be performed after synthesis gas preparation for example, when coal or biomass are used.

According to the present invention, synthesis gas is introduced into an alcohol synthesis unit, together with ethanoic acid to produce a stream comprising methanol and ethanol, in addition to unreacted ethanoic acid. According to a preferred embodiment of the present invention at least a part, preferably all, of said synthesis gas stream is produced by the aforementioned synthesis gas generation process.

Additionally by-products such as methane and higher alcohols may also be produced during alcohol synthesis (i.e. methanol and ethanol synthesis). According to a preferred embodiment of the present invention, the stream exiting the alcohol synthesis unit is subsequently purified to remove said by-products by any methods known to those in the art to obtain substantially pure methanol and ethanol products.

For the targeted production of ethanol the preferred molar ratio, (H₂-CO₂):(CO+ CO₂) (where the concentrations of relevant components are expressed in volume percent or mole percent), of the fresh synthesis gas feed stream fed into the alcohol synthesis unit is greater than 3.0, more preferably greater than 4.0 and most preferably greater than 4.1. Preferably the molar ratio, (H₂-CO₂):( CO+ CO₂), of the fresh synthesis gas feed stream fed into the alcohol synthesis unit is less than 8.5, preferably less than 5.0 and most preferably less than 4.4. The Applicants have also unexpectedly found that the co-feed of CO and CO₂ into the alcohol synthesis unit was particularly beneficial to the selectivity of the process according to the present invention. Therefore CO₂ represents more than 1 vol%, preferably more than 2 vol% and most preferably more than 5 vol% of the total alcohol synthesis reactor gas phase composition.

In order to obtain the aforementioned high synthesis gas molar ratios, additional H₂ needs to be added to the synthesis gas feed stream exiting the synthesis gas generation unit. Preferably, this said H₂ is obtained from the aforementioned synthesis gas generation stage. This is preferably performed by first removing CO₂ and water from the generated synthesis gas followed by a cryogenic separation to isolate the substantially pure CO from the H₂. Alternative methods of separation, such as membrane separation technologies can also be employed. Alternatively, said H₂ stream may also be obtained from another suitable source, such as another chemical process (e.g. off-gas from steel manufacture or electrolysis). Said H₂ stream(s) may still contain inert impurities such as methane, nitrogen, noble gases, water and C₁ to C₄ paraffinic hydrocarbons, which are preferably removed before use.

An added advantage of this aspect of the present invention, when compared to other processes in the field, is that the purification of the separated H₂ feed produced during synthesis gas generation has no requirement for the removal of carbon oxides. Indeed, this is advantageous when compared to a process with a separate hydrogenation stage for ethanoic acid where the carbon oxides act as poisons to the hydrogenation catalyst as well as increasing the formation of inert materials, thus necessitating an increased purge step.

According to a preferred embodiment of the present invention, the alcohol synthesis unit may be any reactor that is suitable for producing methanol and ethanol, for example a fluidised bed reactor or a fixed bed reactor, which can be run with or without external heat exchange equipments e.g. a multi-tubular reactor; or a fluidised bed reactor; or a void reactor.

Preferably the alcohol synthesis unit is operated at a temperature of more than 180 °C, preferably more than 200 °C and most preferably more than 220 °C; and less than 290 °C, preferably less than 280 °C, more preferably less than 270 °C and most preferably less than 250 °C. Preferably the alcohol synthesis unit is operated at pressure of more than 2 MPa and preferably more than 5 MPa; and less than 10 MPa and preferably less than 9MPa. In fact, since methanol synthesis is an exothermic reaction, the chosen temperature of operation is governed by a balance of promoting the forward reaction (i.e. by not adversely affecting the equilibrium) and aiding the rate of conversion (i.e. higher productivity). The ethanol synthesis is also exothermic; however, in this case the chosen temperature of operation is governed by a balance of reaction rate and selectivity.

The GHSV for continuous operation may be in the range 50 to 50,000 h⁻¹, preferably from 1,000 to 30,000 h⁻¹, more preferably from 2,000 to 18,000 h⁻¹ and most preferably from 5,000 to 12,000 h⁻¹.

The ethanoic acid liquid substrate introduced into the alcohol synthesis unit preferably has an LHSV less than 10 h⁻¹, more preferably less than 5 h⁻¹ and most preferably less than 3 h⁻¹; for example, a typical LHSV for normal operation is approximately 1 h⁻¹.

A key feature of the present invention is that the synthesis of methanol from synthesis gas and the hydrogenation of ethanoic acid to ethanol occurs in the same alcohol synthesis unit. Therefore the catalyst for methanol synthesis from synthesis gas and the catalyst for ethanoic acid hydrogenation may be one and the same catalyst; or alternatively more than one catalyst may be employed in the alcohol synthesis unit. The catalyst, or catalysts, may be any suitable catalyst known to those skilled in the art to catalyse the synthesis of methanol from synthesis gas and those which are known to those skilled in the art to catalyse the hydrogenation of carboxylic acids and/or esters to alcohols.

For the avoidance of doubt, when it is hereinafter referred to as a *mixture* of a methanol catalyst and a hydrogenation catalyst, it also covers physical blends of the two catalysts and/or separate packed zones of the two catalysts in the same reactor(s); according to a preferred mode of operation, the hydrogenation catalyst is located downstream of said methanol synthesis catalyst; thus, according to a preferred embodiment of the present invention, the catalyst configuration of the alcohol synthesis unit is such that the stream(s) comprising CO, H₂ and ethanoic acid, is first reacted in the presence of a methanol synthesis catalyst and subsequently reacted in the presence of a hydrogenation catalyst.

The catalyst for the alcohol synthesis unit can be chosen amongst traditional methanol synthesis catalysts selected from one of the two following groups:
i. the high pressure zinc catalysts, composed of zinc oxide and a promoter; and
ii. low pressure copper catalysts, composed of zinc oxide, copper oxide, and a promoter.

A preferred methanol synthesis catalyst is a mixture of copper, zinc oxide, and a promoter such as chromia or alumina. The Applicants have unexpectedly found that the said methanol synthesis catalyst demonstrated high hydrogenation activities.

According to a preferred embodiment of the present invention, the preferred catalyst used in the alcohol synthesis unit is either a hydrogenation catalyst or consists of a mixture of the above methanol catalyst together with a hydrogenation catalyst. The hydrogenation catalyst can be selected from the following:
(i) a precious metal based catalyst, comprising of at least one noble metal from Group VIII of the periodic table (CAS version, for example iron, ruthenium, osmium, cobalt, rhodium, iridium, nickel, palladium, platinum) and at least one of the metals chosen from rhenium, tungsten and/or molybdenum; and optionally an additional metal, that is capable of alloying with said Group VIII noble metal;
(ii) a copper-based catalyst (for example a copper chromite or a mixed copper metal oxide based catalyst wherein the second metal can be copper, zinc, zirconium or manganese), and
(iii) mixtures thereof.

According to a preferred embodiment of the present invention, the hydrogenation catalyst (which may be mixed with the methanol catalyst) is a copper based catalyst, most preferably comprising copper and zinc.

All of the aforementioned hydrogenation catalysts may advantageously be supported on any suitable support known to those skilled in the art; non-limiting examples of such supports include carbon, silica, titania, clays, aluminas, zinc oxide, zirconia and mixed oxides. Preferably, the palladium based catalyst is supported on carbon. Preferably, the copper based catalyst is supported on zinc oxide.

According to an aspect of the present invention, at least a part of the stream exiting the alcohol synthesis unit is passed through a separation unit (e.g. a separation column) to recover and collect a stream comprising the targeted ethanol and a stream comprising methanol.

This separation stage may be performed by any suitable means known to those skilled in the art, e.g. a sieve tray column, a packed column, a bubble cap column or a combination thereof.

Since methyl ethanoate and ethyl ethanoate can also be present in the exit stream from the alcohol synthesis unit, the Applicants have found the following preferred mode of operation(s):
(i) methanol/methyl ethanoate mixture can be easily recovered together with the methanol and fed directly into the downstream carbonylation unit, and/or
(ii) ethanol/ethyl ethanoate mixture can be advantageously recovered and recycled to the alcohol synthesis unit.

Additionally by-products such as methane, ethane and other higher alcohols may also be produced during alcohol synthesis. According to a preferred embodiment of this aspect of the present invention, the streams exiting the separation zone are subsequently purified to remove said by-products from the methanol and ethanol streams by any methods known to those skilled in the art.

According to an aspect of the present invention, at least a part of the aforementioned stream comprising methanol (and optionally methyl ethanoate), together with a substantially pure CO stream, are introduced into a carbonylation reactor. Preferably at least part, most preferably all, of the said methanol stream, emanates from the aforementioned alcohol synthesis unit. However in practice said methanol stream may also emanate from another suitable source, such as a bio-fermentation process and/or pyrolysis (e.g. wood pyrolysis).

Preferably at least a part of the said CO stream is obtained from the aforementioned synthesis gas generation stage. This is preferably performed by first removing CO₂ and water from the generated synthesis gas followed by a cryogenic separation to isolate the substantially pure CO from the H₂. A particular advantage according to the present invention is that both the H₂ fed to the alcohol synthesis unit and the CO fed to the carbonylation unit are obtained from the same synthesis gas separation stage. Alternative methods of separation, such as membrane separation technologies can also be employed. Alternatively, said CO stream may also be obtained from another suitable source, such as another chemical process (e.g. off-gas from steel manufacture). Said substantially pure CO stream may contain inert impurities such as CO₂, methane, nitrogen, noble gases, water and C₁ to C₄ paraffinic hydrocarbons which are preferably removed before use.

According to this aspect of the present invention, the step of introducing methanol, together with CO, into a carbonylation reactor is performed under conditions favourable for producing ethanoic acid.

There are many examples in the prior art which disclose carbonylation processes that can be suitably used in the present invention.

For example, such carbonylation processes can be made in the presence of iridium catalysts as described in US 3772380. UK patent GB 1276326 also describes the preparation of mono-carboxylic acids by carbonylation of alcohols in the presence of rhodium or iridium catalysts, halogen promoters and water or an alcohol, ether or ester.

Carbonylation processes in the presence of ruthenium and osmium catalysts can also be suitably used in the present invention. Thus, UK patents GB 1234641 and GB 1234642 describe a process for the production of an organic acid by carbonylation of an alcohol in the presence of a noble metal catalyst selected from iridium, platinum, palladium, osmium and ruthenium and their compounds and a promoter which is halogen or halogen compound. According to Jenner et al, Journal of Molecular Catalysis, 1987, 40, 71-82 ruthenium compounds are effective carbonylation catalysts for converting primary alcohols into acids at high CO pressures. Standard conditions of 45 MPa CO pressure were used in the reported experiments. For example, UK patent application GB 2029409 describes a process for the preparation of aliphatic carboxylic acids by reacting CO with alcohols at an elevated pressure of 3.4 MPa or greater in the presence of a ruthenium catalyst and halogen-containing promoter.

According to a preferred embodiment of this aspect of the present invention, the carbonylation process takes place in the presence of an iridium catalyst together with at least one promoter; indeed, such catalyst systems have proven to have beneficial effects on the rate of carbonylation of methanol. Said carbonylation process is thus preferably performed in the presence of at least a finite concentration of water with a catalyst system comprising:
(a) an iridium catalyst, (b) methyl iodide and (c) at least one promoter.

Thus, according to a preferred embodiment of this aspect of the present invention the process for the production of ethanoic acid by carbonylation of methanol comprises contacting methanol with CO, in the liquid reaction composition, in a carbonylation reactor wherein, the liquid reaction composition comprises:
(a) ethanoic acid, (b) an iridium catalyst, (c) methyl iodide, (d) water and (e) at least one promoter.

According to an embodiment of this aspect of the present invention, during the carbonylation process, water may be formed in situ in the liquid reaction composition. For example, water may be produced via by-product formation, generated during methane production. Water may also be generated during the esterification reaction between methanol reactant and ethanoic acid product. Water may also be introduced to the carbonylation reactor together with, or separately from, other components of the liquid reaction composition. Water may be separated from other components of reaction composition withdrawn from the reactor and may be recycled in controlled amounts to maintain a preferred concentration of water in the liquid reaction composition. Preferably, the concentration of water in the liquid reaction composition of the carbonylation reactor is in the range 0.1 to 15 wt %, more preferably 1 to 10 wt %, most preferably 1 to 6.5 wt %.

The iridium catalyst in the liquid reaction composition may comprise any iridium containing compound which is soluble in the liquid reaction composition. The iridium catalyst may be added to the liquid reaction composition for the carbonylation reaction in any suitable form which dissolves in the liquid reaction composition or is convertible to a soluble form. Examples of suitable iridium-containing compounds which may be added to the liquid reaction composition include IrCl₃, IrI₃, IrBr₃, [Ir(CO)₂I]₂, [Ir(CO)₂Cl]₂, [Ir(CO)₂Br]₂, [Ir(CO)₂I₂]⁻ H⁺, [Ir(CO)₂Br₂]⁻ H⁺, [Ir(CO)₂I₄]⁻ H⁺, [Ir(CH₃)I₃(CO)₂]⁻H⁺, Ir₄(CO)₁₂, IrCl₃.3H₂O, IrBr₃.3H2O, Ir₄(CO)₁₂, iridium metal, Ir₂O₃, IrO₂, Ir(acac)(CO)₂, Ir(acac)₃, iridium ethanoate, [Ir₃O(OAc)₆(H₂O)₃][OAc], and hexachloroiridic acid [H₂IrCl₆], preferably, chloride-free complexes of iridium such as ethanoates, oxalates and acetoacetates which are soluble in one or more of the carbonylation reaction components such as water, alcohol and/or carboxylic acid. Particularly preferred is green iridium ethanoate which may be used in an ethanoic acid or aqueous ethanoic acid solution.

Preferably, the iridium carbonylation catalyst concentration in the liquid reaction composition is in the range 100 to 6000 ppm by weight of iridium, more preferably 700 to 3000 ppm by weight of iridium.

In this aspect of the present invention at least one promoter is present in the reaction composition. Suitable promoters are preferably selected from the group consisting of ruthenium, osmium, rhenium, cadmium, mercury, zinc, gallium, indium and tungsten, and are more preferably selected from ruthenium and osmium and most preferably is ruthenium. Preferably, the promoter is present in an effective amount up to the limit of its solubility in the liquid reaction composition and/or any liquid process streams recycled to the carbonylation reactor from the ethanoic acid recovery stage. The promoter is suitably present in the liquid reaction composition at a molar ratio of promoter : iridium of [0.5 to 15] : 1. As noted above, the beneficial effect of a promoter such as ruthenium has been found to be greatest at the water concentration which gives the maximum carbonylation rate at any defined methyl ethanoate and methyl iodide concentration. A suitable promoter concentration is 400 to 5000 ppm by weight.

The promoter may comprise any suitable promoter metal-containing compound which is soluble in the liquid reaction composition. The promoter may be added to the liquid reaction composition for the carbonylation reaction in any suitable form which dissolves in the liquid reaction composition or is convertible to soluble form.

Examples of suitable ruthenium-containing compounds which may be used as sources of promoter include ruthenium (III) chloride, ruthenium (III) chloride trihydrate, ruthenium (IV) chloride, ruthenium (III) bromide, ruthenium metal, ruthenium oxides, ruthenium (III) methanoate, [Ru(CO)₃I₃]⁻H⁺, [Ru(CO)₂I₂]ₙ, [Ru(CO)₄I₂], [Ru(CO)₃I₂]₂, tetra(aceto)chlororuthenium(II,III), ruthenium (III) ethanoate, ruthenium (III) propanoate, ruthenium (III) butanoate, ruthenium pentacarbonyl, trirutheniumdodecacarbonyl and mixed ruthenium halocarbonyls such as dichlorotricarbonylruthenium (II) dimer, dibromotricarbonylruthenium (II) dimer, and other organoruthenium complexes such as tetrachlorobis(4-cymene)diruthenium(II), tetrachlorobis(benzene)diruthenium(II), dichloro(cycloocta-1,5-diene)ruthenium (II) polymer and tris(acetylacetonate)ruthenium (III).

Examples of suitable osmium-containing compounds which may be used as sources of promoter include osmium (III) chloride hydrate and anhydrous, osmium metal, osmium tetraoxide, triosmiumdodecacarbonyl, [Oₛ(CO)₄I₂], [Os(CO)₃I₂]₂, [Os(CO)₃I₃]⁻H⁺, pentachloro- mu -nitrodiosmium and mixed osmium halocarbonyls such as tricarbonyldichloroosmium (II) dimer and other organoosmium complexes.

Examples of suitable rhenium-containing compounds which may be used as sources of promoter include Re₂(CO)₁₀, Re(CO)₅Cl, Re(CO)₅Br, Re(CO)₅I, ReCl₃.xH₂O, [Re(CO)₄I]₂, [Re(CO)₄I₂]⁻ H⁺, and ReCl₅.yH₂O.

Examples of suitable cadmium-containing compounds which may be used include Cd(OAc)₂, CdI₂, CdBr₂, CdCl₂, Cd(OH)₂, and cadmium acetylacetonate.

Examples of suitable mercury-containing compounds which may be used as sources of promoter include Hg(OAc)₂, HgI₂, HgBr₂, HgCl₂, Hg₂I₂, and Hg₂Cl₂.

Examples of suitable zinc-containing compounds which may be used as sources of promoter include Zn(OAc)₂, Zn(OH)₂, ZnI₂, ZnBr₂, ZnCl₂, and zinc acetylacetonate. Examples of suitable gallium-containing compounds which may be used as sources of promoter include gallium acetylacetonate, gallium ethanoate, GaCl₃, GaBr₃, GaI₃, Ga₂Cl₄ and Ga(OH)₃.

Examples of suitable indium-containing compounds which may be used as sources of promoter include indium acetylacetonate, indium ethanoate, InCl₃, InBr₃, InI₃, InI and In(OH)₃.

Examples of suitable tungsten-containing compounds which may be used as sources of promoter include W(CO)₆, WCl₄, WCl₆, WBr₅, WI₂, or C₉H₁₂W(CO)₃ and any tungsten chloro-, bromo- or iodo-carbonyl compound.

Preferably, the iridium- and promoter-containing compounds are free of impurities which provide or generate in situ ionic iodides which may inhibit the reaction, for example, alkali or alkaline earth metal or other metal salts.

Ionic contaminants such as, for example, (a) corrosion metals, particularly nickel, iron and chromium and (b) phosphines or nitrogen containing compounds or ligands which may quaternise in situ; should be kept to a minimum in the liquid reaction composition as these will have an adverse effect on the reaction by generating I⁻ in the liquid reaction composition which has an adverse effect on the reaction rate. Some corrosion metal contaminants such as for example molybdenum have been found to be less susceptible to the generation of I⁻. Corrosion metals which have an adverse affect on the reaction rate may be minimised by using suitable corrosion-resistant materials of construction. Similarly, contaminants such as alkali metal iodides, for example lithium iodide, should be kept to a minimum. Corrosion metal and other ionic impurities may be reduced by the use of a suitable ion exchange resin bed to treat the reaction composition, or preferably a catalyst recycle stream. Such a process is described in US 4007130. Preferably, ionic contaminants are kept below a concentration at which they would generate 500 ppm by weight of I⁻, preferably less than 250 ppm by weight of I⁻ in the liquid reaction composition.

Preferably, the concentration of methyl iodide in the liquid reaction composition is in the range 1 to 20 wt %, preferably 5 to 16 wt %.

The partial pressure of CO in the carbonylation reactor is suitably in the range 0.1 to 7 MPa preferably 0.1 to 3:5 MPa and most preferably 0.1 to 1.5 MPa.

The presence of H₂ in the CO feed and generated in situ by the water-gas shift reaction is preferably kept low as its presence may result in the formation of hydrogenation products. Thus, the molar ratio of H₂ to CO reactant is preferably less than 0.01:1, more preferably less than 0.005:1 and yet more preferably less than 0.003:1 and/or the partial pressure of H₂ in the carbonylation reactor is preferably less than 0.1 MPa, more preferably less than 0.05 MPa and yet more preferably less than 0.03 MPa.

The catalyst system used in the carbonylation process of this aspect of the present invention has been found to be particularly beneficial at relatively low partial pressures of CO where the rate of reaction may be dependent upon the CO partial pressure. Under these conditions, it has been found that the catalyst system has the advantage of providing an increased rate of reaction over catalyst systems without the promoters of the present invention. This advantage allows for an increased rate of reaction under conditions when the CO partial pressure is relatively low, for example due to a low total pressure in the carbonylation reactor or due to high vapour pressure of components of the liquid reaction composition, e.g. at high methyl ethanoate concentration in the liquid reaction composition or due to a high concentration of inert gases (for example nitrogen and CO₂ in the carbonylation reactor. The catalyst system may also have advantages of increasing rate of carbonylation when the rate of reaction is reduced by the availability of CO in solution in the liquid reaction composition resulting from mass transfer limitations, for example due to poor agitation.

The pressure of the carbonylation reaction is suitably in the range 0.9 to 19.9 MPa, preferably 0.9 to 9.9 MPa, most preferably 1.4 to 4.9 MPa. The temperature of the carbonylation reaction is suitably in the range 100 to 300 °C, preferably in the range 150 to 220 °C.

Ethanoic acid may advantageously be used as a solvent for said carbonylation reaction.

The carbonylation process of the present invention may be performed as a batch or continuous process, preferably as a continuous process and may be performed in any suitable reactor, known to those skilled in the art.

The ethanoic acid product may be removed from the reactor by withdrawing liquid reaction composition and separating the ethanoic acid product by one or more flash and/or fractional distillation stages from the other components of the liquid reaction composition such as iridium catalyst, ruthenium and/or osmium and/or indium promoter, methyl iodide, water and unconsumed reactants which may be recycled to the reactor to maintain their concentrations in the liquid reaction composition. The ethanoic acid product may also be removed as a vapour from the stream exiting the carbonylation reactor.

Although halide promoters and stabilizers, such as methyl iodide, improve the efficiency and productivity of carbonylation processes, the continued presence of halide compounds in the carbonylation reaction products is undesirable if the product is employed as a starting material in a subsequent process employing a halide-sensitive catalyst where poisoning effects may be cumulative and irreversible. In a preferred embodiment the ethanoic acid product is purified of halide compounds. This purification treatment can be achieved by any appropriate method known to those skilled in the art. For example halides can be removed from the liquid phase using silver salts either unsupported, or supported, on an ion-exchange resin or a zeolite as exemplified in US 5344976 and references therein.

According to a preferred embodiment of the present invention, the separated ethanoic acid from the stream exiting the carbonylation reactor is purified to remove carbonylation catalyst and water, before its introduction into the alcohol synthesis unit. After purification and before introduction into the alcohol synthesis unit, the ethanoic acid stream contains preferably less than 0.1 ppm wt of carbonylation catalyst, more preferably less than 0.05 ppm wt, most preferably less than 0.005 ppm wt.

According to a preferred embodiment of the present invention, at least a part, preferably all, of the said ethanoic acid feed stream originates from the aforementioned carbonylation reaction; however in practice, it may also originate from another suitable source, such as wood pyrolysis and/or as a by-product of a fermentation process to produce alcohol(s).

After purification and before introduction into the alcohol synthesis unit, the ethanoic acid stream contains preferably less than 20 mol % of water.

According to a preferred embodiment of the present invention, water represents between 0.5 and 20 mol %, preferably between 0.5 and 15 mol % and most preferably between 1 and 5 mol % of the total liquid feed (ethanoic acid and water) to the alcohol synthesis unit.

The ethanoic acid fed into the alcohol synthesis unit is preferably purified of sulphur and halide compounds. This purification treatment can be achieved by any appropriate method known to those skilled in the art. For example, halides and iodides can be removed in the liquid phase using silver salts (either homogeneous or supported) on either an ion-exchange resin or a zeolite, or for the removal of sulphides the purification treatment may be performed in the vapour phase, by passing the vapour over a sacrificial bed of zinc oxide (or copper zinc oxides).

Furthermore, the ethanoic acid feed is preferably vapourised prior to contacting the alcohol synthesis catalyst by either, heating the ethanoic acid in a separate vapouriser prior to having contact with the synthesis gas or, by heating the ethanoic acid within the synthesis gas (e.g. either in a separate vessel or on a prebed to the alcohol synthesis reactor). The feed mixture including recycles entering the alcohol synthesis unit (e.g. the synthesis gas, together with the ethanoic acid) is preferably more than 10 °C, preferably more than 20 °C above its dew point temperature.

It should be noted that whilst all of the aforementioned temperature and pressure operating conditions form preferred embodiments of the present invention, they are not, by any means, intended to be limiting, and the present invention hereby includes any other pressure and temperature operating conditions that achieve the same effect.

## Claims

1. A process for producing methanol and ethanol, wherein the methanol is produced from synthesis gas and the ethanol is produced via the hydrogenation of an ethanoic acid feed; **characterised in that** the hydrogenation of the ethanoic acid feed is carried out with the same catalyst(s), and within the same alcohol synthesis unit, that is used to produce the said methanol from the said synthesis gas.

2. A process for the conversion of synthesis gas to ethanol, **characterised by** the following steps:
1) introducing synthesis gas, together with ethanoic acid, into an alcohol synthesis unit to produce methanol and ethanol,
2) separating the methanol from the ethanol of step 1,
3) introducing methanol, from step 2, together with CO, into a carbonylation reactor in the presence of a methanol carbonylation catalyst, to produce ethanoic acid,
4) feeding ethanoic acid, produced in step 3, into the alcohol synthesis unit of step 1, and
5) recovering ethanol from step 2.

3. A process for the conversion of a carbonaceous feedstock(s) into ethanol, **characterised by** the following steps:
1) introducing a carbonaceous feedstock into a synthesis gas reactor to produce a mixture of carbon oxide(s) and H₂,
2) introducing CO and H₂, from step 1, together with ethanoic acid, into an alcohol synthesis unit to produce methanol and ethanol,
3) separating the methanol from the ethanol of step 2,
4) introducing methanol, from step 3, together with CO, into a carbonylation reactor in the presence of a methanol carbonylation catalyst, to produce ethanoic acid,
5) feeding ethanoic acid, produced in step 4, into the alcohol synthesis unit of step 2, and
6) recovering ethanol from step 3.

4. Process according to any of the preceding claims wherein the molar ratio, (H2-CO₂):(CO+ CO₂) (where the concentrations of relevant components are expressed in volume percent or mole percent), of the fresh synthesis gas feed stream fed into the alcohol synthesis unit is greater than 3.0.

5. Process according to any of the preceding claims wherein the molar ratio, (H₂-CO₂):(CO+CO₂), of the fresh synthesis gas feed stream fed into the alcohol synthesis unit is less than 8.5.

6. Process according to any of the preceding claims wherein the alcohol synthesis unit is operated at a temperature of more than 180 °C.

7. Process according to any of the preceding claims wherein the alcohol synthesis unit is operated at a temperature of less than 290 °C.

8. Process according to any of the preceding claims wherein the alcohol synthesis unit is operated at a pressure of more than 2 MPa.

9. Process according to any of the preceding claims wherein the alcohol synthesis unit is operated at a pressure of less than 10 MPa.

10. Process according to any of the preceding claims wherein the alcohol synthesis unit catalyst(s) is a copper based catalyst, most preferably comprising copper and zinc.

11. Process according to claim 9 wherein the alcohol synthesis unit also comprises a hydrogenation catalyst selected from
(i) a precious metal based catalyst, comprising of at least one noble metal from Group VIII of the periodic table (CAS version, for example iron, ruthenium, osmium, cobalt, rhodium, iridium, nickel, palladium, platinum) and at least one of the metals chosen from rhenium, tungsten and/or molybdenum; and optionally an additional metal, that is capable of alloying with said Group VIII noble metal,
(ii) a copper-based catalyst which is different from the methanol catalyst (for example a copper chromite or a mixed copper metal oxide based catalyst wherein the second metal can becopper, zinc, zirconium or manganese), and
(iii) mixtures thereof.

12. Process according to claim 11 wherein the hydrogenation catalyst mixed with the methanol catalyst is a supported copper based catalyst which is different from the methanol catalyst which comprises copper and promoters, such as cobalt and/or manganese and/or chromium, supported on zinc oxide.

13. Process according to either of claims 2 to 12 wherein the process for the production of ethanoic acid by carbonylation of methanol comprises contacting methanol with CO, in the liquid reaction composition, in a carbonylation unit wherein, the liquid reaction composition comprises:
(a) ethanoic acid, (b) an iridium catalyst, (c) methyl iodide, (d) water and (e) at least one promoter.

14. Process according to claim 13 wherein the concentration of water in the liquid reaction composition of the carbonylation unit is in the range 0.1 to 15% by weight, more preferably 1 to 10% by weight, most preferably 1 to 6.5% by weight.

15. Process according to any of claims 13 and 14, wherein the iridium carbonylation catalyst concentration in the liquid reaction composition is in the range 100 to 6000 ppm by weight of iridium, more preferably 700 to 3000 ppm by weight of iridium.

16. Process according to any of claims 13 to 15 wherein the promoter is selected from the group consisting of ruthenium, osmium, rhenium, cadmium, mercury, zinc, gallium, indium and tungsten, and is present in the liquid reaction composition at a molar ratio of promoter : iridium of [0.5 to 15] : 1.

17. Process according to any of claims 13 to 16 wherein the concentration of methyl iodide in the liquid reaction composition is in the range 1 to 20% by weight, preferably 5 to 16% by weight.

18. Process according to any of the preceding claims wherein the ethanoic acid stream is purified to remove, carbonylation catalyst and water, before its introduction into the alcohol synthesis unit.

19. Process according to claim 18 wherein the ethanoic acid stream contains less than 0.1 ppm wt of carbonylation catalyst, preferably less than 0.05 ppm wt, more preferably less than 0.005 ppm wt.

20. Process according to any of claims 18 to 19 wherein the ethanoic acid stream contains preferably less than 20 wt % of water.

21. Process according to any of the preceding claims wherein water represents between 0.5 and 20 mol %, preferably between 0.5 and 15 mol % and most preferably between 1 and 5 mol % of the total liquid feed to the alcohol synthesis unit.

22. Process according to any of the preceding claims wherein the ethanoic acid stream is vapourised prior to its introduction into the alcohol synthesis unit.

23. Process according to any of the preceding claims wherein the feed mixture including recycles entering the alcohol synthesis unit is more than 10 °C, preferably more than 20 °C above its dew point temperature.
